# EUROPEAN PATENT SPECIFICATION

(11) **EP 3 628 244 B1**
(45) Date of publication and mention of the grant of the patent: **18.11.2026**
(21) Application number: 19186610.2
(22) Date of filing: 24.07.2007
(51) Int. Cl.: A61B 17/14, A61B 17/15, A61F 2/44, A61F 2/30, A61F 2/46, A61B 17/16

(54) **INTERVERTEBRAL IMPLANT WITH KEEL**
BANDSCHEIBENIMPLANTAT MIT KIEL
IMPLANT INTERVERTÉBRAL AVEC QUILLE

(30) Priority: 24.07.2006 US 832595 P
(43) Date of publication of application: 01.04.2020
(62) Document number(s) of the earlier application(s) in accordance with Art. 76 EPC: 07813286.7 / 2 043 563
(73) Proprietor: Centinel Spine Schweiz GmbH, 6300 Zug (CH)
(72) Inventor: BERTAGNOLI, Rudolph, 1190 Vienna (AT); HANSON, Shaun, West Chester, PA 19380 (US); RICHTER, Joern, 79400 Kandern (DE); FURDA, John, Hamilton, NJ 08619 (US); HALLER, Fabian, 4053 Basel (CH); GERBER, David, West Chester, PA 19382 (US); BERGER, Roger, 4413 Bueren (CH); KAUFMANN, Stefanie, 4053 Basel (CH)
(74) Representative: Schulz, Oliver Frank Michael

(56) References cited:
- US-A1- 2003 208 273
- US-A1- 2005 159 818
- US-A1- 2005 203 626

## Description

### BACKGROUND OF THE INVENTION

Historically, when it was necessary to completely remove a disc from between adjacent vertebrae, the conventional procedure was to fuse the adjacent vertebrae together. This "spinal fusion" procedure, which is still in use today, is a widely accepted surgical treatment for symptomatic lumbar and cervical degenerative disc disease.

More recently, there have been important developments in the field of disc replacement, namely disc arthoplasty, which involves the insertion of an artificial intervertebral disc implant into the intervertebral space between adjacent vertebrae. Such a disc implant allows limited universal movement of the adjacent vertebrae with respect to each other. The aim of total disc replacement is to remove pain generation (caused by a degenerated disc), restore anatomy (disc height), and maintain mobility in the functional spinal unit so that the spine remains in an adapted sagittal balance. Sagittal balance is defined as the equilibrium of the trunk with the legs and pelvis to maintain harmonious sagittal curves and thus the damping effect of the spine. In contrast with fusion techniques, total disc replacement preserves mobility in the motion segment and mimics physiologic conditions.

One such intervertebral implant includes an upper part that can communicate with an adjacent vertebrae, a lower part that can communicate with an adjacent vertebrae, and an insert located between these two parts. To provide an anchor to the adjacent vertebrae, each part includes a vertically extending keel. Examples of this type of implant are disclosed in U.S. Patent No. 5,314,477 (Marnay), US2005/203626A1 (Sears et al.) and U.S. Patent No. 7,204,852 (Marnay et al.).

While this and other known implants represent improvements in the art of artificial intervertebral implants, there exists a continuing need for improvements for these types of implants.

It will also be noted that in order to provide a keel slot in a vertebra, a cutting of the bone needs to be performed. Typically the cut is made by chiseling, drilling or milling. Combinations of these procedures are possible too. However, where a chisel cut is made using a chisel and a mallet, quite high forces are applied in direction of the cut. With drilling, lesser forces are applied, but the drill can slip of or bend during drilling. With milling, a precise cut is made without high forces, but the milling tool needs to have a certain diameter, because otherwise it will brake during milling so milling is not always possible where a long narrow cut is required. Thus, a procedure used to perform narrow cuts without applying high forces is desirable. Exemplary of such prior art devices and methods are those disclosed in USPA 2004-0215198 (Marnay et al.) and USPA 2006-0064100 (Bertagnoli et al.).

From WO 2006/136760 A1 an intervertebral implant is known having a plurality of anchoring elements on opposing outer surfaces of the implant, wherein the anchoring elements are aligned with each other. Furthermore, US 2004/0117022 A1 discloses intervertebral implants having a single keel at each of the opposing outer surfaces of the implant, wherein the single keels are aligned with each other.

### BRIEF SUMMARY OF THE INVENTION

The scope of the present invention is defined by independent claim 1. Preferred embodiments are depicted in the dependent claims, the description and the Figures. In accordance with a disclosed embodiment, an intervertebral implant includes two components each having an outer surface for engaging an adjacent vertebra and an opposing inner surface. A fixation elements comprising a keel extends from the outer surface of one component and is designed to be disposed in a slot provided in the adjacent vertebra. This keel extends in a plane which is perpendicular to the outer surface and has a leading end in the insertion direction and a trailing end opposite the insertion direction. Furthermore, a pair of fixation elements, preferably a pair of keels, extend on either side of a vertical mid-plane of the other outer surface, which are offset laterally from the fixation element of the first component. The pair of fixation elements or keels are preferably symmetrically located on either side of a vertical mid-plane of the outer surface, and are divergent or convergent with respect to each other.

According to an example, not claimed by the invention, an intervertebral implant component of an intervertebral implant includes an outer surface for engaging an adjacent vertebra and an inner surface. A keel extends from the outer surface and is designed to be disposed in a slot provided in the adjacent vertebra. An anterior shelf is also provided at an anterior end of the outer surface, and this anterior shelf extends vertically away from the inner surface in order to help prevent bone growth from the adjacent vertebra towards the inner surface. The anterior shelf can have a forward surface which is angled, an exterior surface which is polished, and/or a surface treatment which helps prevent bone growth thereon.

Further in accordance with disclosed embodiments, various materials and forms of construction of the component are disclosed. Posterior and/or anterior reductions of the keel are possible for different benefits. The body strength of the vertebra with keel slots on both the superior and inferior surfaces can also be stronger if the keels of the associated components requiring slots are laterally offset. Embodiments of components with modular keels, as well as a variety of advantageous keel shapes (both in horizontal cross section and vertical cross section) are also disclosed.

It will also be appreciated that various combinations of the features disclosed hereafter for a component, and hence for the implant, are also possible as desired.

An instrument for cutting of keel slots with a saw blade, and in particular for cutting multiple slots simultaneously, is also provided as an example, not claimed by the invention.

Other features and advantages of the present invention are stated in or apparent from detailed descriptions of presently preferred embodiments of the inventions found hereinbelow.

### BRIEF DESCRIPTION OF THE SEVERAL VIEWS OF THE DRAWINGS

Figure 1 is a bottom, right and back perspective view of an implant component in accordance with the present invention.
Figure 2 is a top and front perspective view of the implant component depicted in figure 1.
Figure 3 is cross-sectional side elevational perspective view of the implant component of figure 1 taken along the line 3-3 of figure 1.
Figure 4 is an exploded front, bottom, left side perspective view of the implant component of figure 1.
Figure 5 is a bottom and back perspective view of a second embodiment of an implant component in accordance with the present invention.
Figure 6 is a top, front and left side perspective view of the implant component depicted in figure 5.
Figure 7 is cross-sectional side elevational perspective view of the implant component of figure 5 taken along the line 7-7 of figure 5.
Figure 8 is an exploded bottom, right and back perspective view of the implant component of figure 5.
Figure 9 is a top, front and left side perspective view of an implant component with a pointed keel.
Figure 10 is a top, back and right side perspective view of an implant component with an H-shaped keel.
Figure 11 is a top, back and left side perspective view of an implant component inserted in a vertebra with an anterior corner of the keel exposed.
Figure 12 is a top, back and left side perspective view of an implant component with a keel with a rounded top corner.
Figure 13 is a top, back and left side perspective view of an implant component with a keel with a chamfered top corner.
Figure 14 is a top, back and left side view of a vertebra with symmetrically cut slots for centered keels.
Figure 15 is a top and front perspective view of mating implant components with two keels of one component offset from the single keel of the other component.
Figure 16 is top, back and left side view of a vertebra with offset cut slots for the keels of the components depicted in figure 15.
Figure 17 a front view of mating implant components with one keel of one component offset from the other keel of the other component.
Figure 18 is a front and top perspective view of an implant component with two keels that are divergent.
Figure 19 is a front and top perspective view of an implant component with two keels that are convergent.
Figure 20 is a left side view of an implant component having a bone growth retarding plate which is inserted in a vertebra.
Figure 21 is a top plan view of the implant component of figure 20.
Figures 22-24 are top plan views of implant components with different modular keel shapes retainable therein.
Figures 25-31 are top, back and left side perspective views of implant components with differently shaped keels for better longitudinal retention.
Figures 32-51 are front elevation views of different keel shapes.
Figures 52-58 are top plan views of different keel shapes.
Figures 59-61 are schematic front elevation views of mating implant components with different thicknesses of keels.
Figure 62 is a top, back and right side perspective view of an instrument used to cut keel slots.
Figure 63 is a top, front and right side perspective view of the cutting tool depicted in figure 62.
Figure 64 is a top, front and right side perspective view of an alternative cutting tool for use with the instrument depicted in figure 62.

### DETAILED DESCRIPTION OF THE INVENTION

### Materials.

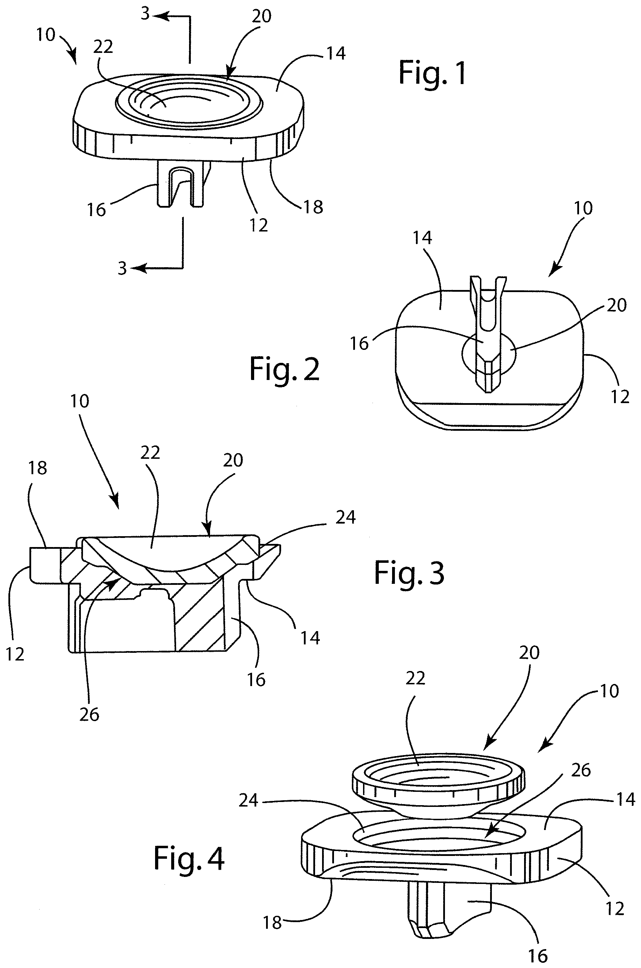

With reference now to the drawings in which like numerals represent like elements throughout the views, a first component 10 of an intervertebral implant for total disc replacement according to the present invention is depicted in Figures 1-4. The implant including component 10 is primarily designed for insertion between adjacent vertebrae from an anterior direction. Thus, reference will sometimes be made to anterior and posterior directions for convenience. However, it will be appreciated that insertion from other directions is possible, and hence the referenced directions would thus be similarly changed. In addition, terms such as front/back, forward/rearward, left/right and top/bottom may be used to identify directions as depicted in the figures and/or as the implant is used relative to any insertion direction, even though the "front" may be facing anteriorly or posteriorly depending on the direction of insertion used. Thus, these terms are used for illustration purposes only and not as limiting terms for the invention.

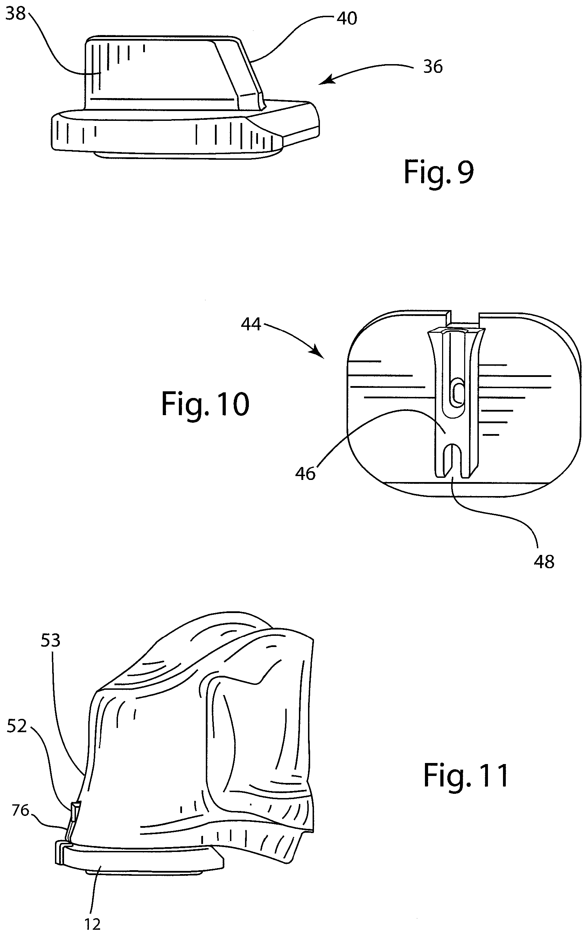

In this embodiment, implant component 10 is formed of an endplate 12 having an outer surface 14 and an integral keel 16 extending outwardly away from outer surface 14. Outer surface 14 is designed to engage an adjacent vertebra, with integral keel 16 then being located in a slot suitably formed in the vertebra (see figure 11 for an illustration of a vertebra having a component mounted therein). Component 10 also includes an inner surface 18 in which an insert 20 is securely located. Insert 20 includes in this embodiment a concavity 22 therein, but it will be appreciated that insert 20 could instead have a convexity. Received in concavity 22 will be a mating component of the implant, allowing a ball joint movement of a similar endplate engaging an adjacent vertebra. The similar endplate could have a mating convexity provided thereon, either integrally formed or as an insert (like insert 20); or alternatively the similar endplate could be part of a substantially identical component, and a third component could be interposed between the two components to provide articulation between the facing concavities (or convexities) of the components.

Component 10 is designed to help overcome the problem of artifacts which arises when an MRI is taken of a metal orthopedic medical device such as an intervertebral implant typically having two such components. During spine surgery, MRI is a standard diagnostic tool used to determine the state of the anatomy by visualizing the soft tissue and nerve roots relative to the bony anatomy. However, commonly used metals for orthopedic devices cause MRI artifacts of different degrees. The amount of imaging artifact is reduced as the density of the material and magnetic properties of the material are decreased. For example, the following biomedical materials create imaging artifacts in decreasing order: stainless steel, CoCr alloy, Titanium alloy, ceramics and plastic polymer materials.

The design of component 10 is made to have a reduced amount of imaging artifact, and is thus comprised of two materials: a soft low density material with properties similar to that of the surrounding bone as the main construct foundation, and a harder dense material insert with superior wear properties for the articulating surface areas or parts. In particular, component 10 includes endplate 12 made of a titanium or titanium alloy, and insert 20 made of a material with good articulating properties such as Co-Cr. It will also be noted that insert 20 is reduced to a small area defining the articulating surface of the implant, further helping to reduce the MRI artifact problem.

Conveniently, insert 20 is a shrink-fit into a cylindrical portion 24 of a receiving and mating cavity 26 of endplate 12 located adjacent inner surface 18, though other ways to secure insert 20 as known in the art are possible (such as a sliding/locking ledge design, threads, adhesives, soldering, pressing, etc.). In this embodiment, it will be appreciated that the receiving cavity 26 of endplate 12 has been optionally designed to pass through outer surface 18, so that a portion of insert 20 is viewable and flat with outer surface 18 as shown in figure 2.

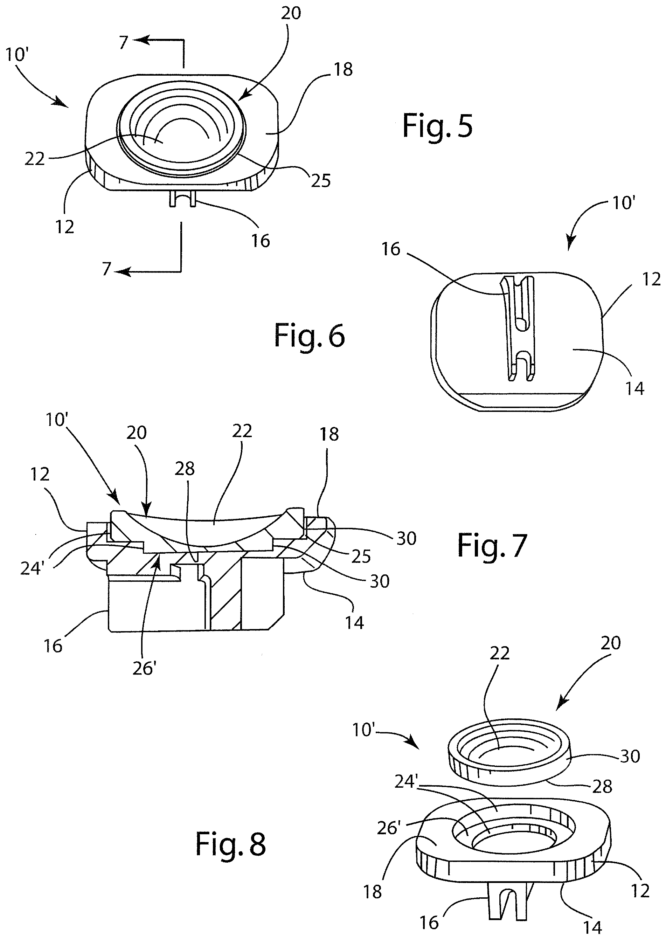

Depicted in figures 5-8 is an alternative embodiment of a component 10' which is broadly similar to component 10 and whose similar elements will thus be identified with the same reference numerals followed by a prime ('). In this embodiment of component 10', insert 20' has a wide flat bottom 28 (as shown best in figure 7, for example) which mates with the similar shape of cavity 26'. This bottom 28 is used to secure insert 20' to endplate 12' by use of a suitable adhesive or the like, while the cylindrical areas 30 on the sides of insert 20' in cavity 26' are slightly spaced from the surrounding metal of cylindrical portion 24' of endplate 12' to create an air channel 25 between the two as shown. It will also be noted that cavity 26' does not extend through to outer surface 14', as shown in figure 6.

Other alternatives to the embodiments above could include any number of hard materials and/or surface treatments to be used for the articulating function, such as a ceramic insert, a titanium nitride coated hardened surface, a diamond coated surface such as a DLC (amorphic diamond like carbon), or any other type surface treatment or material for medical use that provides a hardened superior wear surface. A layer of Co-Cr, ceramic, carbon or other biocompatible low-friction material could also be plasma coated and/or sputtered onto the low-density material of an endplate in a position thereof providing the articulating area (ball and/or socket). This layer of material can then be ground, polished, and/or treated to create the desired low-friction, low-wear articulating surfaces.

Alternatives to the titanium base could be PEEK, PEKK, or some other structural polymer, carbon reinforced or other similar composite materials, or any other low density structural biomaterial. A possible alternative could be a pyrolitic carbon implant with a smooth articulating surface and roughened bone contacting surfaces similar to that used in hand and wrist implants.

### Posterior Keel Reduction.

It has been found that during surgical implantation final seating of the implant endplates has in a few cases proven to be difficult. It is believed that during surgical preparation (typically chiseling, or cutting or drilling) of the keel receiving channel in the vertebral body, not all of the cut bone material is removed but instead some material may be forced to the posterior (or closed) end of the channel by the action of the chisel or the like. This material is then inadvertently left to form an obstruction to the full seating of the keel at the closed end of the channel, resulting in a suboptimal implant position. In order to alleviate this problem, a number of designs are proposed with means designed to accommodate for such excess material, as by a posterior (forward) reduction of the keel. The concept is to create a significantly reduced angled/inclined surface to the forward (or posterior) edge of the keel, more pronounced than the large chamfer at the forward end of the keel such as shown in USP 7,204,852 which is designed instead for easier insertion of the keel.

Thus, a means for accommodating excess material in accordance with the present invention is shown in figure 9. In this embodiment, component 36 has a keel 38 with a front edge formed as a pointed edge 40 (or knife edge shape). With pointed edge 40, keel 38 can easily cleave through any excess material located in the bottom end of the channel in the vertebra. A similar solution would be to reduce the length of the keel by increasing the distance between the posterior (forward) face of the keel and the posterior (bottom) edge of the component. The depth (bottom) of the clearance cut preparation in the bone of the channel for the keel would then remain the same, but the anterior to posterior (or trailing to leading) length of the keel would be reduced on the posterior (forward or leading) end. Doing this would create added clearance between the bone material left in the channel and the posterior (forward) surface of the keel.

Still another means for accommodating excess material is shown in figure 10. In this embodiment, component 44 has a keel 46 with a forward U-shape, and preferably an overall an H-shape in plan view. This U-shape can be provided by removing remove material out of the central forward portion of keel 46 so as to create a slot 48 that goes down the front of keel 46. Any obstructive bone material in the channel would fill into the slot 48 and allow keel 46 to fully seat in the formed channel.

### Anterior Keel Reduction.

It has also been found that in rare cases, due to the irregular anatomy of some patients and/or in cases of extreme anterior (rearward) positioning of the implant, an anterior (rearward) top (outer) corner 52 of a keel may be proud or protruding out from the anterior (rearward) surface 54 of the bone as shown in figure 11. This exposed metal corner can cause irritation in surrounding tissue. For that reason, a means for slightly reducing the anterior (rearward) corner profile in the keel in the anterior (rearward) end (as well as posterior end, if desired, as noted above) is desirable.

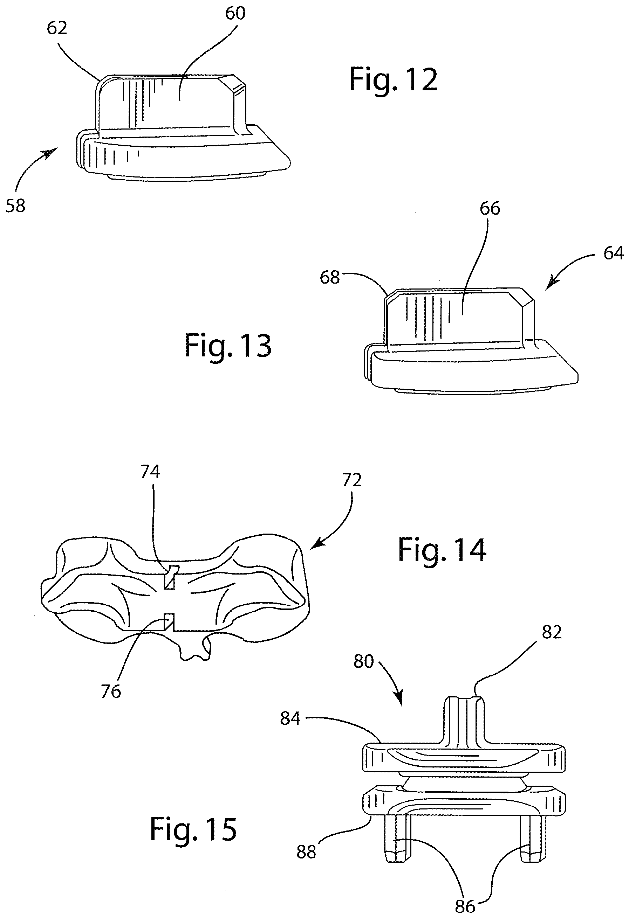

One means for slightly reducing the anterior (rearward) corner profile in the keel at the anterior end is to provide a component 58 with a keel 60 in which the anterior (rearward) corner is reduced by a curved rounded surface 62 as shown in figure 12. Another means for slightly reducing the anterior (rearward) corner profile in the keel at the anterior (rearward) end is to provide a component 64 with a keel 66 in which the anterior (rearward) corner is reduced by a chamfer 68 as shown in figure 13.

Other embodiments could include an angled surface or other feature to reduce keel material in this area.

### Vertebral Body Strength.

In small or weakened vertebrae, weakening of the bone due to the alignment in the central axis of the spine of keel receiving channels 74 and 76 superior and inferior to vertebra 72 in a multi-level (consecutive) disc replacement case will occur as shown in figure 14. This occurrence would leave a much smaller central portion of the bone in vertebra 72, possibly creating a weaker bony construct surrounding the two keels of the two adjacent implants. Thus, it was determined that designs where the keels would be offset in different locations would be desirable for leaving a more intact stronger bone construct.

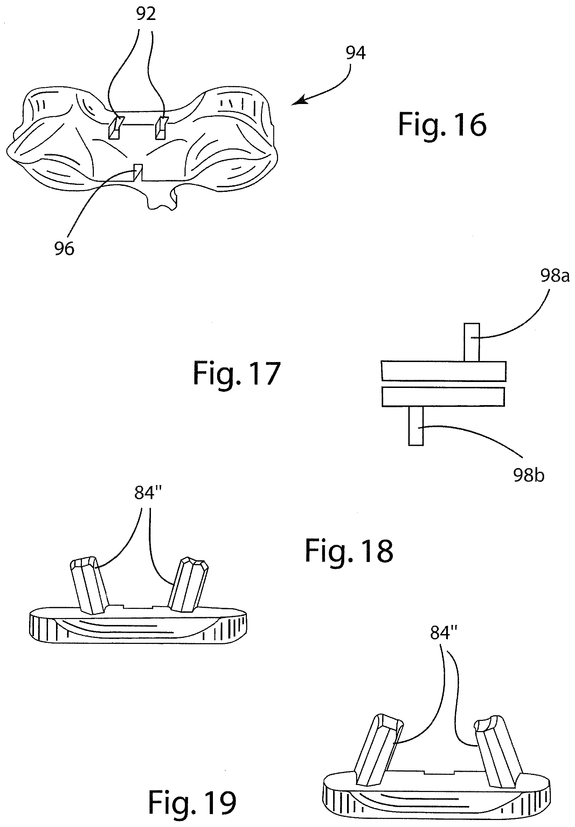

One means for offsetting keels in adjacent implants is depicted in figures 15-16. In this embodiment, an implant 80 has a single central keel 82 on one (superior) endplate 84 and double laterally offset keels 86 on the other (inferior) endplate 88. Obviously, the positions of double keels could instead be superior, if desired. However, the desired result of a stronger vertebra is as shown in figure 16 where such an implant is used both above and below the depicted vertebra. In particular, there are double keel receiving channels 90 in the superior side of vertebra 92 which are on either side (laterally offset) from single keel receiving channel 94 in the inferior side of vertebra 92 - leaving a larger amount of bone in the central area of vertebra 92.

As a variation of this design, the two components of an implant could have oppositely offset keels 98a and 98b as depicted in figure 17.

Another variations of this design could include embodiments wherein offset keels 84' are divergent at a certain angle as shown in figure 18, or offset keels 84" are convergent as shown in figure 19. The enhanced benefit of having divergent/convergent keel designs is to prevent loosening of the associated endplate with that type of geometry. The divergent or convergent angles of the keels would add greater resistance to movement in the axial direction and become less likely to be loosened over time. Thus, it will be appreciated that such divergent or convergent keel designs would be beneficial even when not used in consecutive implants; and thus the keels of paired endplates could be both convergent or both divergent, or one convergent and one divergent.

### Prevention of Fusion.

It has been found that in some cases of total disc arthroplasty in the neck, bone has grown across the implant located in between the vertebral bodies so that the adjacent vertebrae have become fused in spite of the articulating implant provided therebetween. Mostly this problem occurs in the anterior portion of the implant. Therefore, in order to prevent this from happening, a means is added to the implant endplates to retard or stop bone from bridging over the implant.

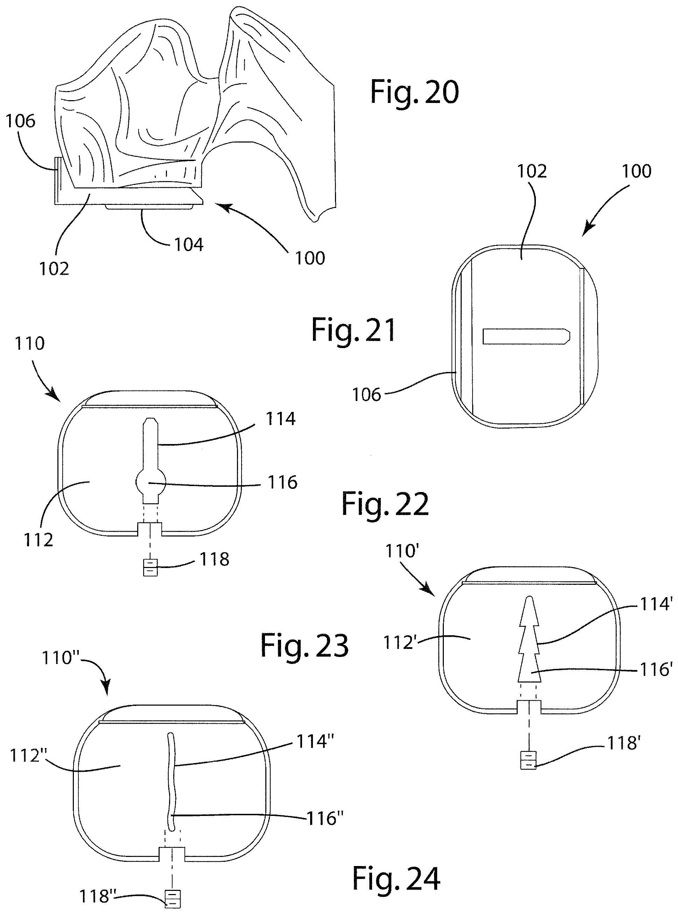

Depicted in figures 20-21 is an implant component 100 having an endplate 102 and insert 104. Provided at the anterior (outer) end of component 102 is a raised plate edge or shelf-like feature 106. The presence of raised edge 106 serves as a means for retarding bone growth/formation between the adjacent vertebrae by its presence and large distance which must then be bridged.

It will also be appreciated that tissue, including bone tissue, tends to grow into and anchor to rough surfaces of titanium implants but does not adhere to certain plastics or other materials. Thus, this raised anterior edge 106 of endplate 104 is preferably polished to a smooth surface finish. Alternatively, the raised anterior edge 106 is treated with a suitable surface coating since bone fusion is usually related to the blood supply and cell formation/cell growth for a given area. For example, an anti-cellular coating could be placed in this area to prevent bone forming and hence undesirable bone fusion. Alternatively, an anti-blood coagulating surface or agent could be integral to raised edge 106. Raised edge 106 could also be designed to hold cement or other material that would contain an anticoagulant or anti-cellular growth inhibiting agent. The bone cement, implant coating, and/or implant edge fusion block feature could also contain a controlled release anti-inflammatory agent to retard the healing process and thus retard bone growth in that area of undesired fusion.

### Modular Keels.

In order to improve fixation of a keel while avoiding removing of some of the vertebra, the keel or other fixation feature of an implant could also be modular as depicted in figures 22-24. With such an embodiment, the endplate is placed into the intervertebral space first; and then the fixation element, such as a keel, is moved through the plate into a mating slot provided in the bone. Finally, that fixation element is then locked to the plate by a suitable mechanical means such as a fastener or set screw. Thus, depicted in figure 22 is an implant component 110 having an endplate 112 in which there is a central aperture 114 shaped to matingly receive a keel 116. As shown, set screw 118 is used to secure keel 116 in place in aperture 114 of endplate 112 once keel 116 is located properly. Keel 116 has a "ball" shape at one end, with the vertebra thus having a cutout or slot designed to receive this ball shape. With this shape, keel 116 is more stably held in place longitudinally, without removing so much of the vertebrae.

Other possible keel geometries or configurations are possible, such as the "Xmas Tree" shape of keel 116' of component 110' depicted in figure 23, or the snake-shaped keel 116" of component 110" depicted in figure 24. It would also be alternatively possible to put the keel in place first, and then attach the endplate to the keel in a similar manner.

### Keels Shapes.

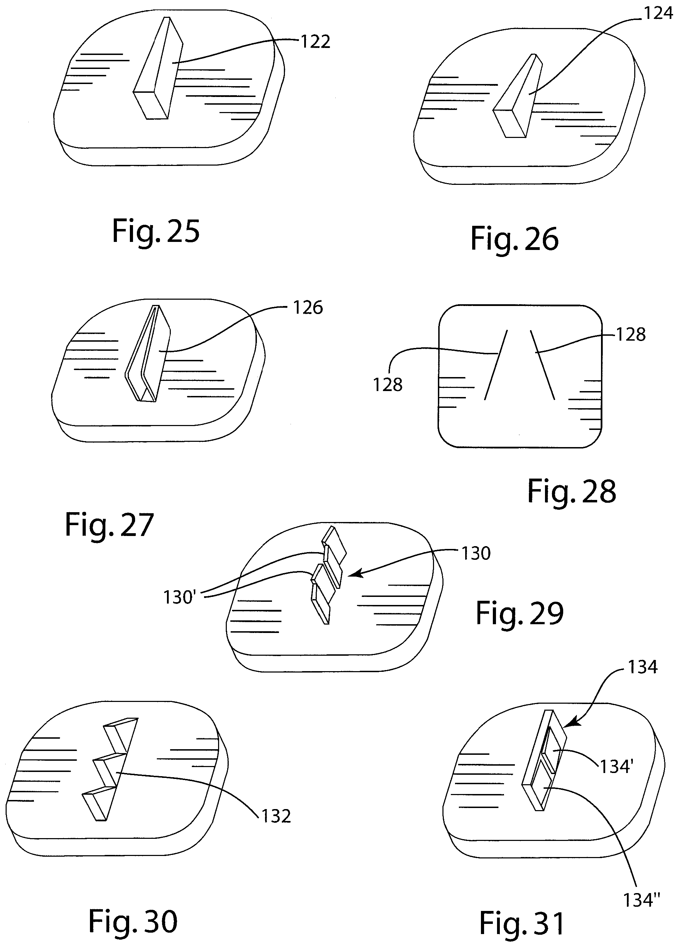

A variety of different keel shapes are also possible to enhance the ability of the keel to be retained (or not to loosen as easily) in the slot cut into the vertebra. For example, the shape of the keel can be slightly wedged in one dimension (in the forward or insertion direction) as shown by keel 122 in figure 25 (or keel 126 in figure 27); or in two dimensions as shown by keel 124 in figure 26. Typically the wedge shape is regular and symmetrical, but it could also be irregular and unsymmetrical. The wedge shape can go over the whole length of the keel or just in a short section of the keel. The wedge shape of the keel does not need to be solid, and thus could be hollow as shown by keel 126 in figure 27. In this embodiment, keel 126 is open from the top, and from the anterior (outer) end; and in another embodiment the keel could also be open from the front (forward end) as shown schematically by keels 128 in figure 28. Keel 126 or 128 could also be open from the sides as desired; and it will be thus appreciated that combinations of different openings are also possible.

A keel with steps is also desirable, as shown by keels 130, 132 and 134 shown in respective figures 29, 30 and 31. In the case of keel 130, it consists of respective small parts 130' that are grouped together to build the keel-shape. It will be appreciated that keel 134 also provides special surfaces preventing the backing out by the keel, namely angled out fin or surface 134' and angled in surface 134", even though keel 134 is easy to insert in one (forward) direction as surface 134' has a ramping action in that direction.

While the vertical cross-sectional shape of a keel is typically a simple rectangular shape, the vertical cross-sectional geometry of a keel could also be modified to enhance fixation and/or stability in the bone. This cross section can vary, as it can be symmetrical or asymmetrical. Some examples of different vertical cross-sectional shapes, and combinations of vertical cross-sectional shapes, are shown in the drawings as described hereafter with reference to the noted figures.

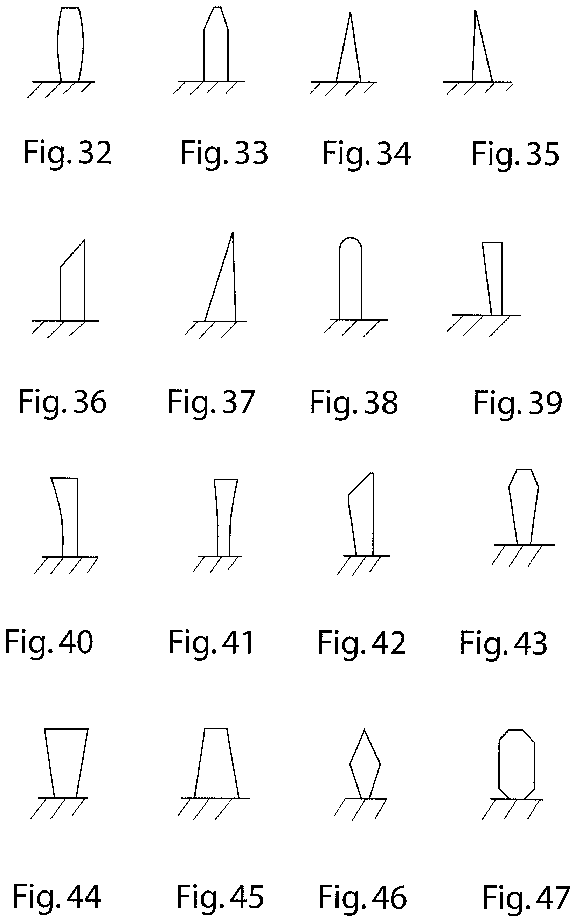

- Figure 32:: bowed on each side;
- Figure 33:: angled asymmetrically on each top side;
- Figure 34:: pointed at the top side;
- Figure 35:: angled to one side;
- Figure 36:: top edge angled;
- Figure 37:: right angle triangle shaped;
- Figure 38:: round nosed;
- Figure 39:: one side inwardly angled;
- Figure 40:: one side inwardly bowed;
- Figure 41:: both sides inwardly bowed;
- Figure 42:: top edge angled to one side which is angled inward;
- Figure 43:: angled on each top side, and each side angled inward;
- Figure 44:: both sides angled inward;
- Figure 45:: both sides angled outward;
- Figure 46:: diamond shaped;
- Figure 47:: both ends chamfered;

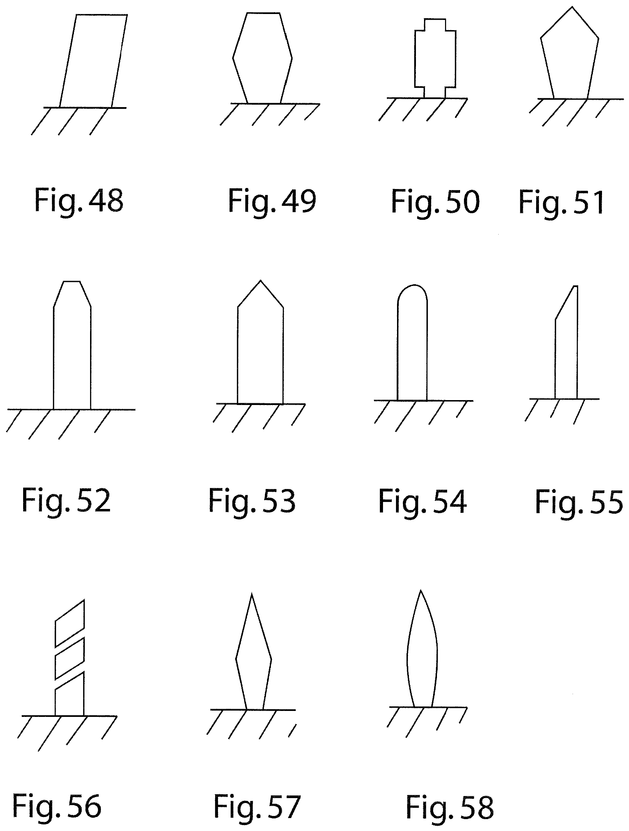

- Figure 48:: parallelogram shaped;
- Figure 49:: elongated hexagonal shaped;
- Figure 50:: both ends stepped at both sides; and
- Figure 51:: elongated pentagon shaped.

Also, while the horizontal cross-sectional shape of a keel is also typically a simple rectangular shape, the horizontal cross-sectional geometry of a keel could also be modified to enhance fixation and/or stability in the bone. This cross section can vary, as it can be symmetrical or asymmetrical. Some examples of different horizontal cross-sectional shapes, besides those already mentioned above, and combinations of horizontal cross-sectional shapes, are shown in the drawings as described hereafter with reference to the following figures.
- Figure 52:: angled symmetrically toward the leading edge;
- Figure 53:: angled symmetrically toward the leading edge to a point;
- Figure 54:: rounded leading edge;
- Figure 55:: sharply angled to one side leading edge;
- Figure 56:: a series of angled leading edges;
- Figure 57:: diamond shaped with trailing edge blunted; and
- Figure 58:: asymmetrically bowed.

Further, where more than one keel is present on one of the two components the thicknesses of the keels can vary. Some examples of differing thickness keels are shown in the drawings as described hereafter with reference to the following figures.

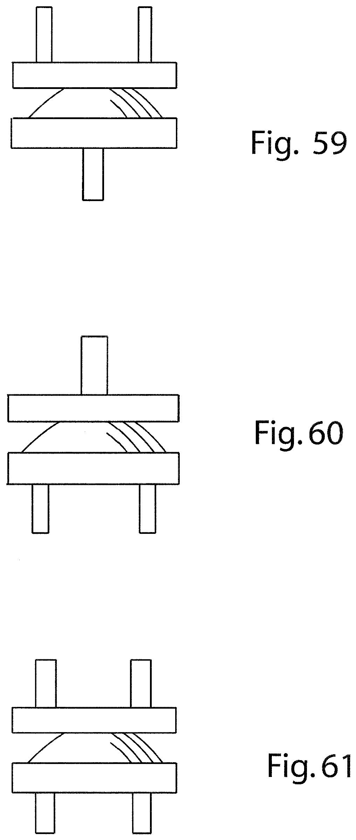

- Figure 59:: the top keels have a small and a large thickness, while the bottom keel has an intermediate thickness;
- Figure 60:: the bottom keels have a small and an intermediate thickness, while the top keel has a large thickness;
- Figure 61:: the top keels have a small and a large thickness, while the bottom keels have a small intermediate thickness and a large intermediate thickness.

### Cutting of Dual Keel Slots.

As noted above, instruments and methods have been disclosed for cutting keel receiving slots in a vertebra, or in two adjacent vertebrae. Typical of such devices and methods are those shown and described in USPA 2004-0215198 (Marnay et al.) and USPA 2006-0064100 Bertagnoli et al.) which primarily disclose chiseling or burring embodiments.

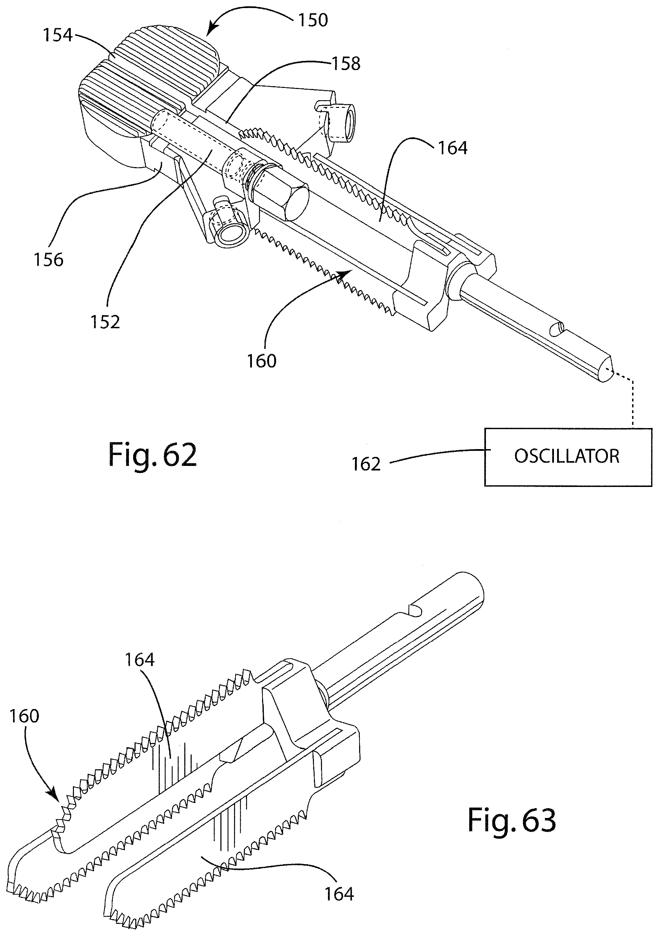

Depicted in figures 62-63 is an instrument broadly similar to those disclosed in the above noted published applications, and thus including a trial implant 150 having an adjustable stop 152. The trial implant 150 includes a top slot 154 at the location above which a keel slot is to be cut in a superior (or inferior) vertebra when trial implant 150 is located between two vertebrae. It will be noted that trial implant also has two bottom slots (not shown) similar to top slot 150, but at locations where offset keel slots are to be cut in the inferior vertebra - so that trial implant 150 is thus used to cut the slots for an implant such as disclosed in figure 15.

Extending away from trial implant 150 is a guide 156 which is used to guide trial implant into the intervertebral space between two adjacent vertebrae after the disc is removed and to which adjustable stop 152 is threadedly engaged. Guide 156 has slots corresponding to those in trial implant 150, such as top slot 158. Slots 154 and 158 serve to guide saw cutting tool 160 therealong, where cutting tool 160 is rapidly reciprocated by a suitable motor 162 shown schematically and which can take the form of various power tools as known in the art. Rapid reciprocation of saw cutting tool 160 is effective to produce is a reduced impact on the vertebral bone due to the acceleration to mass relationship between cutting tool 160 and the vertebral bone.

It will be appreciated that cutting tool 160 includes three thin saw blades 162 which extend at a proximal edge thereof along slots in guide 156 and trial implant 150, such as slots 158 and 154. At the distal edge, saw blades 162 have suitable cutting teeth 164, which at a leading or forward end form a ramp for easier starting into the vertebra. The insertion depth of cutting blades into the vertebrae is controlled by adjusting the position of adjustable stop 152.

While cutting tool 160 has been shown with three blades, it will be appreciated that only a single blade could be provided to cut each slot individually as needed. It would also be possible to provide a blade more like a chisel but with cutting teeth just at the front. Different interchangeable blades would also be possible, if a narrower or wider, or higher or lower, or deeper or shallower, cut slot was desired. If desired, motor 162 can be dispensed with, and the blade or blades moved by hand with the same guidance. The material of the blades is preferably a suitable metal, but ceramic or plastic blades, or even a diamond cutting blade, would also be possible. If desired or necessary, a coatings to reduce friction could be used with the cutting blades.

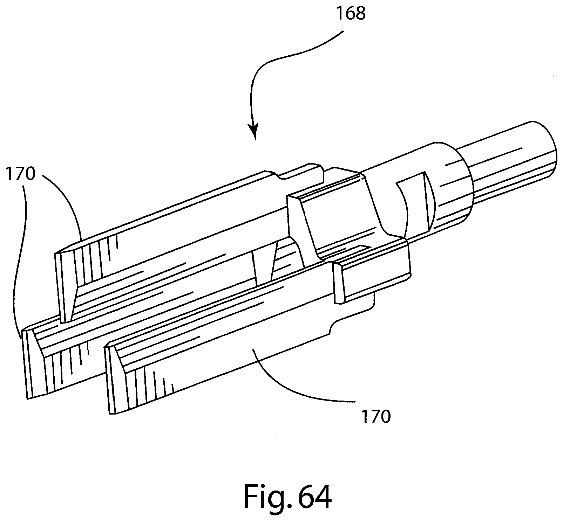

Depicted in figure 64 is another cutting tool 168 usable in place of cutting tool 160 and with trial implant 150. Cutting tool 168 includes chisel blades 170 and would thus be used to chisel three slots simultaneously.

Various advantageous features have been described above with respect to various embodiments. Such advantageous features are also considered to be usable together, rather than singly as typically depicted and described.

While the present invention has been described with respect to exemplary embodiments thereof, it will be understood by those of ordinary skill in the art that variations and modifications can be effected within the scope of the invention.

## Claims

1. An intervertebral implant (80) for insertion into an intervertebral disc space between two adjacent vertebrae along an insertion direction, the implant (80) comprising:
a first component (84) having an outer surface for engaging one of the adjacent vertebrae and an inner surface opposite the outer surface;
a fixation element (82) extending from the outer surface of the first component (84), said fixation element (82) extending in a plane which is substantially perpendicular to said outer surface and having a leading end in the insertion direction and a trailing end opposite the insertion direction, wherein the fixation element (82) comprises a keel; and
a second component (88) having an outer surface for engaging the other of the two adjacent vertebrae and an inner surface opposite the outer surface,
**characterized in that**
the implant (80) comprises a pair of fixation elements (86) extending from the outer surface of the second component (88), said pair of fixation elements (86) extending on either side of a vertical mid-plane of said outer surface, wherein each of the pair of fixation elements (86) of the second component (88) is offset from the fixation element (82) of the first component (84) in the lateral direction.

2. The implant of claim 1, wherein the fixation element (82) includes opposed side walls that are substantially parallel to each other and elongate along the insertion direction, wherein the side walls flare away from each other along a direction opposite the insertion direction so as to define the trailing end.

3. The implant of claim 1, wherein the fixation element (82) is integral with the first component (84).

4. The implant of claim 1, wherein the fixation element (82) extends from the outer surface on a vertical mid-plane of the outer surface.

5. The implant of claim 1, wherein the fixation element (82) of the first component (84) is centered with respect to the pair of fixation elements (86) of the second component (88).

6. The implant of claim 1, further including an insert (20) residing between the first and second components (84, 88).

7. The implant of claim 6, wherein the first component (84) includes a concave surface, and the insert (20) includes a convex surface for engaging the concave surface of the first component (84).

8. The implant of claim 1, wherein the pair of fixation elements (86) of the second component (88) each have opposed side walls that are substantially parallel to each other and elongate along the insertion direction, wherein the opposed side walls flare away from each other along a direction opposite the insertion direction so as to define a second trailing end.

## Patentansprüche

1. Zwischenwirbelimplantat (80) zum Einsetzen in einen Bandscheibenraum zwischen zwei benachbarten Wirbeln entlang einer Einsetzrichtung, wobei das Implantat (80) Folgendes umfasst:
eine erste Komponente (84) mit einer Außenfläche für einen Eingriff mit einem der benachbarten Wirbel und einer der Außenfläche gegenüberliegenden Innenfläche;
ein Befestigungselement (82), das sich von der Außenfläche der ersten Komponente (84) erstreckt, wobei sich das Befestigungselement (82) in einer Ebene erstreckt, die im Wesentlichen senkrecht zu der Außenfläche verläuft, und ein vorderes Ende in der Einsetzrichtung und ein hinteres Ende entgegen der Einsetzrichtung aufweist, wobei das Befestigungselement (82) einen Kiel umfasst; und
eine zweite Komponente (88) mit einer Außenfläche für einen Eingriff mit dem anderen der beiden benachbarten Wirbel und einer der Außenfläche gegenüberliegenden Innenfläche,
**dadurch gekennzeichnet, dass**
das Implantat (80) zwei Befestigungselemente (86) umfasst, die sich von der Außenfläche der zweiten Komponente (88) erstrecken, wobei das Paar Befestigungselemente (86) sich auf beiden Seiten einer vertikalen Mittelebene der Außenfläche erstreckt, wobei jedes der zwei Befestigungselemente (86) der zweiten Komponente (88) gegenüber dem Befestigungselement (82) der ersten Komponente (84) in der seitlichen Richtung versetzt ist.

2. Implantat nach Anspruch 1, wobei das Befestigungselement (82) gegenüberliegende Seitenwände beinhaltet, die im Wesentlichen parallel zueinander verlaufen und sich in der Einsetzrichtung erstrecken, wobei sich die Seitenwände in einer der Einsetzrichtung entgegengesetzten Richtung voneinander wegspreizen, um das hintere Ende zu definieren.

3. Implantat nach Anspruch 1, wobei das Befestigungselement (82) mit der ersten Komponente (84) einstückig ist.

4. Implantat nach Anspruch 1, wobei sich das Befestigungselement (82) von der Außenfläche auf einer vertikalen Mittelebene der Außenfläche erstreckt.

5. Implantat nach Anspruch 1, wobei das Befestigungselement (82) der ersten Komponente (84) in Bezug auf die zwei Befestigungselemente (86) der zweiten Komponente (88) zentriert ist.

6. Implantat nach Anspruch 1, ferner beinhaltend einen Einsatz (20), der sich zwischen der ersten und der zweiten Komponente (84, 88) befindet.

7. Implantat nach Anspruch 6, wobei die erste Komponente (84) eine konkave Oberfläche beinhaltet und der Einsatz (20) eine konvexe Oberfläche für einen Eingriff mit der konkaven Oberfläche der ersten Komponente (84) beinhaltet.

8. Implantat nach Anspruch 1, wobei die zwei Befestigungselemente (86) der zweiten Komponente (88) jeweils gegenüberliegende Seitenwände aufweisen, die im Wesentlichen parallel zueinander verlaufen und sich in der Einsetzrichtung ausdehnen, wobei sich die gegenüberliegenden Seitenwände in einer der Einsetzrichtung entgegengesetzten Richtung voneinander wegspreizen, um ein zweites hinteres Ende zu definieren.

## Revendications

1. Implant intervertébral (80) destiné à être inséré dans un espace discal intervertébral entre deux vertèbres adjacentes le long d'une direction d'insertion, l'implant (80) comprenant :
un premier composant (84) présentant une surface extérieure destinée à se mettre en prise avec l'une des vertèbres adjacentes et une surface intérieure opposée à la surface extérieure ;
un élément de fixation (82) s'étendant depuis la surface extérieure du premier composant (84), ledit élément de fixation (82) s'étendant dans un plan sensiblement perpendiculaire à ladite surface extérieure et présentant une extrémité avant dans la direction d'insertion et une extrémité arrière opposée à la direction d'insertion, dans lequel l'élément de fixation (82) comprend une quille ; et
un deuxième composant (88) présentant une surface extérieure destinée à se mettre en prise avec l'autre des deux vertèbres adjacentes et une surface intérieure opposée à la surface extérieure,
**caractérisé en ce que**
l'implant (80) comprend une paire d'éléments de fixation (86) s'étendant à partir de la surface extérieure du deuxième composant (88), ladite paire d'éléments de fixation (86) s'étendant de chaque côté d'un plan médian vertical de ladite surface extérieure, dans lequel chacun de la paire d'éléments de fixation (86) du deuxième composant (88) est décalé par rapport à l'élément de fixation (82) du premier composant (84) dans la direction latérale.

2. Implant selon la revendication 1, dans lequel l'élément de fixation (82) inclut des parois latérales opposées qui sont sensiblement parallèles l'une à l'autre et s'allongent dans le sens de l'insertion, dans lequel les parois latérales s'évasent l'une de l'autre le long d'une direction opposée à la direction d'insertion afin de définir l'extrémité arrière.

3. Implant selon la revendication 1, dans lequel l'élément de fixation (82) est d'un seul tenant avec le premier composant (84).

4. Implant selon la revendication 1, dans lequel l'élément de fixation (82) s'étend de la surface extérieure sur un plan médian vertical de la surface extérieure.

5. Implant selon la revendication 1, dans lequel l'élément de fixation (82) du premier composant (84) est centré par rapport à la paire d'éléments de fixation (86) du deuxième composant (88).

6. Implant selon la revendication 1, incluant en outre un insert (20) situé entre le premier et le deuxième composants (84, 88).

7. Implant selon la revendication 6, dans lequel le premier composant (84) inclut une surface concave, et l'insert (20) inclut une surface convexe destinée à se mettre en prise avec la surface concave du premier composant (84).

8. Implant selon la revendication 1, dans lequel la paire d'éléments de fixation (86) du deuxième composant (88) présentent chacun des parois latérales opposées qui sont sensiblement parallèles l'une à l'autre et s'allongent le long de la direction d'insertion, dans lequel les parois latérales opposées s'évasent l'une de l'autre dans une direction opposée à la direction d'insertion de manière à définir une deuxième extrémité arrière.
